# EUROPÄISCHE PATENTSCHRIFT

(11) **EP 0 589 341 B1**
(45) Veröffentlichungstag und Bekanntmachung des Hinweises auf die Patenterteilung: **02.01.1997**
(21) Anmeldenummer: 93114814.2
(22) Anmeldetag: 15.09.1993
(51) Int. Cl.: C07C 209/16, C07C 211/38

(54) **Verfahren zur Herstellung von endo/exo-8-N,N-Dialkylaminoexo-tricyclo(5.2.1.0 2,6) decan**
Process for the preparation von endo/exo-8-N,N-dialkylaminoexo-tricyclo(5.2.1.0 2,6) decane
Procédé pour la préparation d'endo/exo-8-N,N-dialkylaminoexo-tricyclo(5.2.1.0 2,6) decane

(30) Priorität: 25.09.1992 DE 4232097
(43) Veröffentlichungstag der Anmeldung: 30.03.1994
(73) Patentinhaber: HOECHST AKTIENGESELLSCHAFT, 65926 Frankfurt am Main (DE)
(72) Erfinder: Dämbkes, Georg, Dr., Dipl.-Chem., D-46537 Dinslaken (DE); Frohning, Carl Dieter, Dr. Dipl.-Chem., D-46495 Wesel (DE); Kniep, Claus, D-46049 Oberhausen (DE); Kampmann, Detlef, Dr. Dipl.-Chem., D-86368 Gersthofen (DE); Weber, Jürgen, Dr. Dipl.-Chem., D-46147 Oberhausen (DE)

(56) Entgegenhaltungen:
- WO-A-90/04567
- JOURNAL OF THE AMERICAN CHEMICAL SOCIETY. Bd. 81 , 1959 , GASTON, PA US Seiten 655 - 658 P. WILDER '1,2-DIHYDRO-ENDO-DICYCLOPENTADIENE'

## Beschreibung

Die Erfindung betrifft ein Verfahren zur Herstellung von endo/exo-8-N,N-Dialkylamino-exo-tricyclo [5.2.1.0^{2,6}] decan aus der entsprechenden 8-Hydroxyverbindung. Quarternäre Ammoniumsalze des Diaminotricyclo [5.2.1.0^{2,6}] decans finden u.a. bei der Herstellung künstlicher Zeolithe als sogenannte Template Anwendung, d.h. als molekulare Negativformen, die das Mineralgerüst während der Kristallisation stabilisieren.

Die Herstellung von 8-N,N-Dialkyamino-tricyclo [5.2.1.0^{2,6}] decan ist bekannt. P. Wilder et al (J. Am. Chem. Soc. 81 (1959), 655 ff) stellen diese Verbindung aus dem Oxim des 8-Ketotricyclo [5.2.1.0^{2,6}] decan her, das mit Natrium zum 8-Aminotricyclo [5.2.1.0^{2,6}] decan reduziert wird. Durch Umsetzung des primären Amins mit einer Mischung aus Ameisensäure und einer wäßrigen Formaldehydlösung erhält man schließlich die Dimethylverbindung in mäßiger Ausbeute.

Auch das in der WO 90/04567 beschriebene Verfahren geht zur Gewinnung von 8-N,N-Dialkylamino-tricyclo [5.2.1.0^{2,6}] decan vom 8-Ketotricyclo [5.2.1.0^{2,6}] decan aus. Es wird in einem geschlossenen System bei 160 bis 195°C in Gegenwart von Ameisensäure mit Dialkylformamid (wobei Alkyl einen Methyl-, Ethyl- oder Propylrest bedeutet) umgesetzt. Die Reaktionszeit beträgt 10 bis 50 Stunden.

Die bekannten Synthesen erfüllen noch nicht alle Ansprüche, die an ein Verfahren gestellt werden, das in technischem Umfang ausgeübt werden soll. Sie erfordern den Einsatz teurer Chemikalien, lange Reaktionszeiten, die die Wirtschaftlichkeit des Prozesses beeinträchtigen und liefern das gewünschte Produkt nur in mäßiger Ausbeute.

Es bestand daher die Aufgabe ein Verfahren zu entwickeln, das die beschriebenen Nachteile vermeidet und technisch einfach und unter Verwendung preiswerter Ausgangsstoffe zu der gewünschten Dialkylaminoverbindung führt.

Diese Aufgabe wird gelöst durch ein Verfahren zur Herstellung von endo/exo-8-N,N-Dialkylamino-exo-tricyclo [5.2.1.0^{2,6}] decan. Es ist dadurch gekennzeichnet, daß 8-Hydroxytricyclo [5.2.1.0^{2,6}] decan bei erhöhter Temperatur in Gegenwart eines Hydrierkatalysators und von Wasserstoff mit einem sekundären Amin der allgemeinen Formel HNR¹R², wobei R¹ und R² gleich oder verschieden sind und jeweils einen Alkylrest mit 1 bis 6 Kohlenstoffatomen bedeuten, unter Abtrennung des bei der Reaktion entstehenden Wassers umgesetzt wird.

Das neue Verfahren ist überraschend selektiv und ergibt endo/exo-8-N,N-Dialkylamino-exo-tricyclo [5.2.1.0^{2,6}] decan in hoher Ausbeute; die Aminoverbindung läßt sich aus dem Reaktionsgemisch durch einfache Destillation in reiner Form gewinnen.

Das als Ausgangsverbindung eingesetzte 8-Hydroxytricyclo [5.2.1.0^{2,6}] decan ist eine marktgängige Substanz, die aus Tricyclo [5.2.1.0^{2,6}] deca-3,8-dien (Cyclopentadien) durch Wasseranlagerung in Gegenwart saurer Katalysatoren und anschließende Hydrierung des Hydroxytricyclodecens hergestellt wird. Sie kann in der handelsüblichen Form, also z.B. ohne vorherige Feinreinigung, angewendet werden.

Erfindungsgemäß läßt man das Hydroxytricyclodecan mit einem sekundären Amin der allgemeinen Formel HNR¹R² reagieren. R¹ und R² können gleich oder verschieden sein. Sie stehen für Alkylreste mit 1 bis 6, vorzugsweise 1 bis 4 und insbesondere 1 bis 3 Kohlenstoffatomen. Geeignete Amine sind Dimethylamin, Diethylamin, Di-n-propylamin, Di-n-butylamin, Di-i-butylamin, Di-n-pentylamin, Di-3-methylbutylamin, Di-n-hexylamin, Di-i-hexylamin, N-Methylethylamin, N-Methylpropylamin, N-Methylbutylamin, N-Methylpentylamin, N-Methylhexylamin, N-Ethylpropylamin, N-Ethylbutylamin, N-Ethylpentylamin, N-Ethylhexylamin, N-Propylbutylamin, N-Propylpentylamin, N-Propylhexylamin, N-Butylpentylamin, N-Butylhexylamin, insbesondere Dimethylamin, Diethylamin,,Di-n-propylamin, bevorzugt Dimethylamin.

Üblicherweise setzt man die Reaktionspartner in äquimolaren Mengen um; jedoch ist es auch möglich, einen der Reaktionspartner im Überschuß anzuwenden.

Die Reaktion zwischen Hydroxyverbindung und sekundärem Amin erfolgt in Gegenwart von Katalysatoren. Obgleich es sich bei der Umsetzung nicht um eine Hydrierung handelt und Wasserstoff an der Reaktion zwischen den Ausgangsstoffen nicht unmittelbar teilnimmt, haben sich herkömmliche Hydrierkatalysatoren, z.B. solche auf Basis von Nickel und Kobalt bei der Durchführung der Reaktion bewährt. Mit besonderem Erfolg setzt man Kupferkatalysatoren ein, die, unerwartet, überaus selektiv wirken. Geeignet sind vorzugsweise Kupferträgerkatalysatoren mit einem Kupfergehalt von 15 bis 85, insbesondere von 50 bis 70 Gew.% (bezogen auf den Katalysator). Als Träger enthalten sie Kieselsäure, Kieselgur, Aluminiumoxid, Magnesiumoxid, Kohle oder Bims, bevorzugt Kieselgur oder Aluminiumoxid und gegebenenfalls übliche Aktivatoren und Zusatzstoffe wie Magnesium oder Chrom. Die Katalysatormenge richtet sich nach dem Anteil der Hydroxyverbindung im Reaktionsgemisch. Üblicherweise wendet man 0,5 bis 30, insbesondere 3 bis 20 Gew.-% Katalysator, bezogen auf die Hydroxyverbindung, an. Um die Katalysatoren zu aktivieren und ihre Aktivität über die gesamte Reaktionszeit zu erhalten, führt man dem Reaktionsgemisch kontinuierlich Wasserstoff in einer Menge von 5 bis 300, insbesondere von 20 bis 100 1/1 Katalysator zu.

Die Umsetzung zwischen den Reaktanten verläuft bei Temperaturen zwischen 120 und 250, insbesondere 145 bis 195°C, vorzugsweise unter Normaldruck, wenngleich auch geringer Überdruck bis zu 0,1 MPa, insbesondere bis zu 0,05 MPa angewendet werden kann. Nach einer bewährten Ausführungsform wird das sekundäre Amin der gerührten Suspension aus 8-Hydroxytricyclo [5.2.1.0^{2,6}] decan und dem Katalysator zugesetzt. Sowohl Amin als auch Tricyclodecan können als Lösung eingesetzt werden, z.B. unter Verwendung von Toluol, Xylol als Lösungsmittel. Um sicherzustellen, daß die Reaktion schnell und vollständig abläuft, ist es erforderlich, für eine kontinuierliche Abtrennung des bei der Umsetzung entstehenden Wassers zu sorgen. Sie erfolgt nach den in der chemischen Technik bekannten Verfahren, z.B. mit Hilfe eines Wasserabscheiders, Phasentrenners oder durch azeotrope Destillation. Die genannten Reaktionsbedingungen stellen sicher, daß bevorzugt das endo/exo-Isomere gebildet wird.

Die neue Arbeitsweise kann sowohl kontinuierlich als auch insbesondere diskontinuierlich durchgeführt werden.

Aus dem Reaktionsgemisch wird das endo/exo-8-N,N-Dialkylamino-exo-tricyclo [5.2.1.0^{2,6}] decan durch einfache Destillation abgetrennt und dabei in so reiner Form gewonnen, daß eine weitere Reinigung für die meisten Anwendungszwecke nicht mehr erforderlich ist.

In den nachfolgenden Beispielen wird die Herstellung von endo/exo-8-N,N-Dimethylamino-exo-tricyclo [5.2.1.0^{2,6}] decan nach dem neuen Verfahren näher erläutert. Es ist selbstverständlich nicht beabsichtigt, die Erfindung auf diese speziellen Ausführungsformen zu beschränken.

### Beispiel 1: Herstellung von endo/exo-8-N,N-Dimethylamino-exo-tricyclo [5.2.1.0^{2,6}] decan

In einem mit Rührer, Thermometer, Wasserabscheider, Rückflußkühler und Einleitungsrohr versehenen 4 1-Dreihalskolben werden 2280 g (15 mol) 8-Hydroxy-tricyclo [5.2.1.0^{2,6}] decan und 228 g eines Kupfer-Trägerkatalysators (RCH 60/35; Handelsprodukt der Hoechst AG, Cu-Gehalt etwa 60 Gew.-%, bezogen auf die Katalysatormasse) vorgelegt und unter Rühren aufgeheizt. Nach Erreichen einer Temperatur von 100°C werden über das Einleitungsrohr fortlaufend 20 bis 25 l/h Wasserstoff zur Aktivierung des Katalysators zugeführt und gleichzeitig über das gleiche Einleitungsrohr 125 g/h (2,78 mol) Dimethylamin gasförmig eingespeist.

Bei einer Temperatur von 148 bis 150°C wird erstmals Wasser im Abscheider aufgefangen. Zur Beschleunigung der Reaktion wird die Temperatur auf 170 bis 175°C eingestellt und die kontinuierliche Zugabe von Wasserstoff und Dimethylamin fortgesetzt. Das gebildete Reaktionswasser wird ständig über den Abscheider entfernt. Nach 6 h wird kein Wasseraustrag mehr beobachtet. Insgesamt werden 328 g Reaktionswasser erhalten, in ihm ist ein Teil des überschüssigen Dimethylamins gelöst.

Nach Abkühlen wird das Reaktionsprodukt filtriert und gaschromatographisch untersucht. Das Rohamin enthält noch 2,98 % restliches 8-Hydroxy-tricyclo [5.2.1.0^{2,6}] decan, das entspricht einem Umsatz von etwa 97 %.

Die Selektivität zu endo/exo-8-N,N-Dimethylamino-exo-tricyclo [5.2.1.0^{2,6}] decan beträgt 80,8 %, das Verhältnis endo- zu exo-Verbindung 61 : 39.

### Beispiel 2

Beispiel 1 wird wiederholt mit dem Unterschied, daß die Reaktionstemperatur 160 bis 165°C beträgt. Das gebildete Reaktionswasser wird wiederum ständig über den Wasserabscheider aus dem Reaktionsgemisch entfernt. Nach 6 h wird kein Wasseraustrag mehr beobachtet. Insgesamt werden 312 g Reaktionswasser erhalten, in dem ein Teil des überschüssigen Dimethylamins gelöst ist. Im abgekühlten und filtrierten Rohamin sind nach gaschromatographischer Bestimmung noch 3,75 % 8-Hydroxy-tricyclo [5.2.1.0^{2,6}] decan enthalten, das entspricht einem Umsatz von etwa 96,2 %.

Die Selektivität zu endo/exo-8-N,N-Dimethylamino-exo-tricyclo [5.2.1.0^{2,6}] decan beträgt 80,2 %, das Verhältnis endo- zu exo-Verbindung 81 : 19.

### Beispiel 3

Beispiel 1 wird wiederholt mit dem Unterschied, daß die Reaktionstemperatur 150 bis 155°C beträgt. Das gebildete Reaktionswasser wird wiederum ständig über den Abscheider aus dem Reaktionsgemisch entfernt. Nach 6 3/4 h wird kein Wasseraustrag mehr beobachtet. Insgesamt werden 284 g Reaktionswasser erhalten, in dem ein Teil des überschüssigen Dimethylamins gelöst ist. Im abgekühlten und filtrierten Rohamin sind nach gaschromatographischer Untersuchung noch 8,84 % 8-Hydroxy-tricyclo [5.2.1.0^{2,6}] decan enthalten, das entspricht einem Umsatz von etwa 91,2 %.

Die Selektivität zu endo/exo-8-N,N-Dimethylamino-exo-tricyclo [5.2.1.0^{2,6}] decan beträgt 86,1 %, das Verhältnis endo- zu exo-Verbindung 95 : 5.

## Patentansprüche

1. Verfahren zur Herstellung von endo/exo-8-N,N-Dialkylamino-exo-tricyclo [5.2.1.0^{2,6}] decan, dadurch gekennzeichnet, daß man 8-Hydroxytricyclo [5.2.1.0^{2,6}] decan bei erhöhter Temperatur in Gegenwart eines Hydrierkatalysators und von Wasserstoff mit einem sekundären Amin der allgemeinen Formel HNR¹R², wobei R¹ und R² gleich oder verschieden sind und jeweils einen Alkylrest mit 1 bis 6 Kohlenstoffatomen bedeuten, unter Abtrennung des bei der Umsetzung entstehenden Wassers umsetzt.

2. Verfahren nach Anspruch 1, dadurch gekennzeichnet, daß R¹ und R² für einen Alkylrest mit 1 bis 4, insbesondere 1 bis 3 Kohlenstoffatomen stehen.

3. Verfahren nach Anspruch 1 oder 2, dadurch gekennzeichnet, daß als Amin Dimethylamin eingesetzt wird.

4. Verfahren nach einem oder mehreren der Ansprüche 1 bis 3, dadurch gekennzeichnet, daß man die Umsetzung bei 120 bis 250°C, vorzugsweise 145 bis 195°C durchführt.

5. Verfahren nach einem oder mehreren der Ansprüche 1 bis 4, dadurch gekennzeichnet, daß man als Hydrierkatalysator einen Kupferträgerkatalysator mit einem Kupfergehalt von 15 bis 85, insbesondere 50 bis 70 Gew.-%, bezogen auf den Katalysator, verwendet.

6. Verfahren nach Anspruch 5, dadurch gekennzeichnet, daß der Katalysator als Trägermaterial Kieselgur oder Aluminiumoxid enthält.

7. Verfahren nach einem oder mehreren der Ansprüche 1 bis 6, dadurch gekennzeichnet, daß man 0,5 bis 30, insbesondere 3 bis 20 Gew.-% Katalysator, bezogen auf die Hydroxyverbindung, anwendet.

8. Verfahren nach einem oder mehreren der Ansprüche 1 bis 7, dadurch gekennzeichnet, daß man dem Reaktionsgemisch kontinuierlich Wasserstoff in einer Menge von 5 bis 300, insbesondere 20 bis 100 l/l Katalysator zuführt.

## Claims

1. Process for the preparation of endo/exo-8-N,N-dialkylamino-exo-tricyclo[5.2.1.0^{2,6}]decane, which comprises reacting 8-hydroxytricyclo[5.2.1.0^{2,6}]-decane with a secondary amine of the formula HNR¹R², in which R¹ and R² are identical or different and are in each case an alkyl radical having 1 to 6 carbon atoms, at elevated temperature in the presence of a hydrogenation catalyst and hydrogen, the water formed during the reaction being removed.

2. The process as claimed in claim 1, wherein R¹ and R² are an alkyl radical having 1 to 4, in particular 1 to 3, carbon atoms.

3. The process as claimed in claim 1 or 2, wherein dimethylamine is employed as the amine.

4. The process as claimed in one or more of claims 1 to 3, wherein the reaction is carried out at 120 to 250°C, preferably 145 to 195°C.

5. The process as claimed in one or more of claims 1 to 4, wherein a copper supported catalyst with a copper content of 15 to 85, in particular 50 to 70% by weight, based on the catalyst, is used as the hydrogenation catalyst.

6. The process as claimed in claim 5, wherein the catalyst comprises kieselgubr or aluminum oxide as the support material.

7. The process as claimed in one or more of claims 1 to 6, wherein 0.5 to 30, in particular 3 to 20% by weight of catalyst, based on the hydroxy compound, is used.

8. The process as claimed in one or more of claims 1 to 7, wherein hydrogen is fed continuously to the reaction mixture in an amount of 5 to 300, in particular 20 to 100 l/l of catalyst.

## Revendications

1. Procédé pour la préparation d'endo/exo-8-N,N-dialkylamino-exotricyclo[5.2.1.0^{2,6}]décane, caractérisé en ce qu'on fait réagir du 8-hydroxytricyclo[5.2.1.0^{2,6}]décane à température élevée en présence d'un catalyseur d'hydrogénation et d'hydrogène avec une amine secondaire de la formule générale HNR¹R², dans laquelle R¹ et R² sont identiques ou différents et représentent à chaque fois un résidu alkyle en C₁-C₆, avec séparation de l'eau produite lors de la réaction.

2. Procédé selon la revendication 1, caractérisé en ce que R¹ et R² représentent un résidu alkyle en C₁-C₄, en particulier en C₁-C₃.

3. Procédé selon la revendication 1 ou 2, caractérisé en ce qu'on utilise en tant qu'amine de la diméthylamine.

4. Procédé selon l'une ou plusieurs quelconques des revendications 1 à 3, caractérisé en ce qu'on réalise la réaction à une température de 120 à 250°C, de préférence de 145 à 195°C.

5. Procédé selon l'une ou plusieurs quelconques des revendications 1 à 4, caractérisé en ce qu'on utilise en tant que catalyseur d'hydrogénation un catalyseur de cuivre à support avec une teneur en cuivre de 15 à 85, en particulier de 50 à 70 % en poids, rapportés au catalyseur.

6. Procédé selon la revendication 5, caractérisé en ce que le catalyseur contient en tant que matériau de support de la diatomite ou de l'oxyde d'aluminium.

7. Procédé selon l'une ou plusieurs quelconques des revendications 1 à 6, caractérisé en ce qu'on utilise de 0,5 à 30, en particulier de 3 à 20 % en poids de catalyseur, rapportés au composé hydroxy.

8. Procédé selon l'une ou plusieurs quelconques des revendications 1 à 7, caractérisé en ce qu'on ajoute en continu au mélange réactionnel de l'hydrogène dans une quantité de 5 à 300, en particulier de 20 à 100 l/l de catalyseur.
